# FASCICULE DE BREVET EUROPEEN

(11) **EP 3 675 653 B1**
(45) Date de publication et mention de la délivrance du brevet: **13.03.2024**
(21) Numéro de dépôt: 18804417.6
(22) Date de dépôt: 31.10.2018
(51) Int. Cl.: A23L 3/3463, A23K 30/00, A61P 31/10, A61K 35/742, A01N 63/22, A01P 3/00

(54) **COMPOSITION ANTIMYCOTOXINE**
ANTIMYKOTOXIN-ZUSAMMENSETZUNG
ANTIMYCOTOXIN COMPOSITION

(30) Priorité: 31.10.2017 FR 1771150
(43) Date de publication de la demande: 08.07.2020
(73) Titulaire: MIXSCIENCE, 35170 Bruz (FR)
(72) Inventeur: LAYUS, Michel, 22560 Pleumeur Bodou (FR); BRAME, Alexandre, 35000 Rennes (FR)
(74) Mandataire: LLR
(86) Numéro de dépôt international: PCT/IB2018/058559
(87) Numéro de publication internationale: WO 2019/087110

(56) Documents cités:
- WO-A1-2013/178947
- US-A1- 2013 045 185
- XIN GAO ET AL: "Isolation of: screening for aflatoxins B, M, and Gdetoxification", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 232, no. 6, 2 avril 2011 (2011-04-02) , pages 957-962, XP019907770, ISSN: 1438-2385, DOI: 10.1007/S00217-011-1463-3

## Description

L'invention concerne une composition antimycotoxine.

Les mycotoxines sont des toxines élaborées par diverses espèces de champignons microscopiques telles que les moisissures (Aspergillus sp., Fusarium sp., Stachybotrys sp., Penicillium sp., etc.). Ce sont des molécules de faible poids moléculaire (< 1 000 daltons), le plus souvent thermostables en milieu non aqueux. Difficilement dégradables, elles peuvent subsister dans les denrées alimentaires même après l'élimination des moisissures.

Les mycotoxines sont considérées comme l'une des principales menaces pour le secteur de l'alimentation et de l'élevage dans le monde entrainant des pertes de productivité animale se situant entre 2 et 5%.

La prévention de la contamination des matières premières par des mycotoxines peut consister en l'utilisation de fongicides inhibant la croissance des moisissures, ou la sélection génétique de plantes résistantes à l'invasion. À cela s'ajoutent les soins apportés lors du stockage (séchage, contrôle de la température, de l'humidité et de l'oxygénation dans les silos) :
- les méthodes physiques : lavage, séchage, broyage, tris manuels ou mécanisés des gousses ou des amandes, séparation mécanique de la coque et de la peau qui sont le lieu essentiel de contamination, traitement par choc thermique, torréfaction...
- les méthodes chimiques : traitement à l'ammoniaque des tourteaux d'arachides. La détoxification par l'ammoniac sous pression se prête bien au traitement des tourteaux d'arachides ou d'autres oléagineux qui arrivent par bateau ;
- les méthodes biologiques comme l'addition d'inhibiteur de moisissures (propionate) ou comme la dilution (amalgame ou mélange) de grains contaminés avec des grains non contaminés pour l'alimentation animale (interdite dans certains pays).

Mais, en l'état actuel des connaissances scientifiques et techniques et ce malgré les améliorations apportées aux techniques de production et de stockage, on ne sait pas empêcher complètement le développement des moisissures. Il est probable que cela ne soit pas possible sans employer des moyens ayant plus d'effets secondaires négatifs, notamment sur le plan écologique, mais aussi sur le plan de la santé. En conséquence, la présence de mycotoxines dans les denrées alimentaires ne peut être totalement éliminée. Par denrées alimentaires, on entend ici l'ensemble des denrées incluant les matières premières, les aliments destinés à l'homme et les aliments destinés aux animaux. Cette présence est par ailleurs fortement dépendante des conditions climatiques, et donc variable selon les années.

D'autre part, il n'est, à ce jour, pas possible d'éliminer les mycotoxines au niveau de la préparation des denrées alimentaires sans altérer la valeur nutritive des produits.

La seule prévention possible est donc d'écarter de la chaîne alimentaire les aliments « trop » contaminés. En fixant le « trop » au niveau adéquat, ce qui n'a rien d'évident entre les réactions des producteurs (considérant les normes comme toujours trop dures) et les exigences sécuritaires (normes toujours trop tolérantes). Sachant que plus le niveau d'exigence est élevé, plus les coûts augmentent (tests, isolement, élimination ou recyclage par des filières non alimentaires...) et moins le bénéfice sanitaire est sensible (par rapport à un niveau d'exigence plus faible mais déjà efficace).

Dans l'Union européenne, les normes concernant les mycotoxines les plus courantes sont fixées par le Règlement 1881/2006 portant fixation de teneurs maximales pour certains contaminants dans les denrées alimentaires et la Directive 2002/32/CE sur les substances indésirables dans les aliments pour animaux.

Parallèlement, la filière de l'élevage et notamment l'industrie des aliments pour animaux sont de plus en plus considérées comme ayant un rôle à jouer pour assurer l'utilisation responsable des antimicrobiens en production animale. Les décideurs demandent aux producteurs d'aliments pour animaux, aux agriculteurs, aux vétérinaires et aux organismes de réglementation de travailler ensemble pour déterminer les meilleures pratiques d'élevage et d'hygiène et des alternatives viables, afin de réduire l'utilisation d'antibiotiques chez les animaux d'élevage et améliorer le bien-être animal. La lutte contre les mycotoxines participe donc à ce projet de filière en luttant contre les pathologies liées aux mycotoxines et donc, indirectement à la baisse des volumes d'antimicrobiens utilisés pour traiter ces pathologies.

Aussi existe-t-il un réel besoin de fournir un moyen d'éliminer les mycotoxines.

En élevage, les pratiques actuelles d'atténuation du risque de mycotoxines sont multiples, et plusieurs solutions sont disponibles sur le marché, telles que les liants (binders).

Certaines sont trop peu sélectives, et peuvent donc vite être saturées par les matrices alimentaires sur lesquelles elles sont appliquées. De même, celles-ci peuvent interagir avec des nutriments ou composés autres que les mycotoxines cibles, en particulier des vitamines ou minéraux. Dans ce cas, la biodisponibilité de ces nutriments en est réduite.

D'autres sont au contraire trop sélectives, et n'apportent une solution que pour une mycotoxine. Il faut donc employer des stratégies combinant plusieurs solutions pour éliminer plusieurs mycotoxines présentes dans un même aliment.

Peu de données sont disponibles à ce jour sur l'efficacité des solutions du marché sur des aliments contaminés en-dessous des niveaux réglementaires en mycotoxines.

Les liants ont notamment pour inconvénient connu que les mycotoxines restent toujours présentes dans l'aliment et donc dans le tube digestif de l'animal, même si liées, et peuvent donc se désorber avec la solution proposée. Les liants peuvent également relarguer des composés toxiques accumulés tels que métaux lourds, dioxines, ...

Enfin, en présence d'une contamination importante en mycotoxines, la croissance de certains microorganimes peut être altérée voire même inhibée, rallongeant ainsi le temps nécessaire pour atteindre un niveau de décontamination satisfaisant.

Les stratégies actuelles de lutte contre les mycotoxines nécessitent donc de cumuler un panel de solutions pour éliminer les mycotoxines et pallier à ces inconvénients.

On connaît de l'état de la technique la demande de brevet US2015306154, qui enseigne l'utilisation de bactéries du genre *Bacillus* pour lutter contre les effets des mycotoxines. Toutefois, ce document enseigne la biotransformation de certaines mycotoxines testées et ne semble pas fournir des résultats satisfaisants sur toutes les mycotoxines.

On connaît également de l'art antérieur la demande WO2013/178947 qui décrit les souches NOL01, NOL02, NOL03, NOL04, NOL11 et NOL12 pour le traitement des colibacilloses, notamment chez la volaille.

On connaît en outre l'enseignement de Xin Gao et al., European Food Research and Technology, Vol 232, n°6, 2 avril 2011, pages 957-962, qui décrit une souche de Bacillus subtilis, la souche ANSB060 capable de détoxifier les milieux contaminés par les aflatoxines B1, M1 et G1.

Aussi, le besoin de fournir une nouvelle méthode reste entier.

L'un des buts de l'invention est de pallier ces inconvénients.

Un autre but de l'invention est de proposer une nouvelle composition capable de se débarrasser définitivement des mycotoxines contenues dans les denrées alimentaires et ainsi améliorer la santé animale.

Aussi, propose-t-on l'utilisation notamment *in vitro* ou *ex vivo* d'une composition comprenant ou consistant essentiellement en
- NOL01, NOL02 et NOL03, lesdites souches étant déposées à la CNCM sous les numéros respectifs CNCM I - 4606, CNCM I-5043, CNCM I - 4607 et CNCM I - 4608, pour la dégradation d'au moins une mycotoxine.

L'invention repose sur la constatation surprenante faite par les inventeurs que des souches spécifiques de bactéries du genre *Bacillus subtilis* sont capables de dégrader plusieurs types de mycotoxines produites par les champignons dans le milieu, sans toutefois produire de résidus toxiques issus de cette dégradation Il est également observé par les inventeurs que cette décontamination est effective sur une gamme large de pH, température, concentration en mycotoxines et aussi bien en condition aérobie qu'anaérobie. Dans l'invention il est donc utilisé les souches NOL01, NOL02 et NOL03.

Par souche de bactéries, on entend dans l'invention l'ensemble des individus (bactéries) issus de repiquages successifs d'une colonie bactérienne.

En d'autres termes, une souche est une partie d'une espèce bactérienne différente des autres bactéries de la même espèce par une différence mineure mais identifiable. Une souche est également définie comme une population de bactéries qui descend d'un seul organisme ou de la culture isolat pur. Les souches d'une même espèce peuvent différer légèrement de l'autre à bien des égards.

La composition susmentionnée comprend la souche NOL03, déposée à la CNCM sous le numéro CNCM I - 4607, et les souches suivantes :
- La souche NOL01 déposée à la CNCM sous le numéro CNCM I - 4606, et
- La souche NOL02 déposée à la CNCM sous le numéro CNCM I - 5043,

Cela signifie que l'invention couvre l'utilisation des combinaisons de souches suivantes :
- NOL01, NOL02, NOL03 et NOL04 la souche NOL04 étant déposée à la CNCM sous le numéro CNCM I - 4608.

Toutes ces souches ont été déposées à la collection nationale des microorganismes (CNCM) à l'Institut Pasteur à Paris, selon le traité de Budapest.

Par dégradation, on entend dans l'invention la destruction des mycotoxines, c'est à dire leur déstructuration par rupture des liaisons covalentes entre tout ou partie des atomes qui les constituent de sorte que le produit final de la dégradation est une ou plusieurs autres molécules ne possédant plus ou substantiellement plus les propriétés toxiques pour les animaux des mycotoxines dont elles sont issues.

L'avantage de la composition selon l'invention est qu'elle est capable d'une part de dégrader et non capter les mycotoxines, mais elle est également capable de dégrader plusieurs mycotoxines de nature différente, c'est-à-dire ayant une structure ou une formule chimique différente.

Dans l'invention on entend par *in vitro et ex vivo* une utilisation qui n'a pas lieu au sein d'un organisme animal supérieur (comme les oiseaux, les mammifères ou les poissons) ou humain, mais n'exclut pas la possibilité d'une utilisation sur des microorganismes, des végétaux ou des champignons.

Avantageusement, l'invention concerne l'utilisation susmentionnée, pour la dégradation d'au moins deux mycotoxines de structures différentes.

L'objectif de la composition selon l'invention est de permettre en une seule utilisation de pouvoir éliminer d'un environnement déterminé non pas une seule mycotoxine, mais deux, voire 3, 4, 5, 6, 7, 8 ou, 9 ou 10 ou plus, mycotoxines de structures ou de formules chimiques différentes.

Plus avantageusement, l'invention concerne l'utilisation susmentionnée, où ladite composition comprend de 10⁴ à 10¹¹ colonies bactériennes, les colonies bactériennes étant par mL ou g de composition.

En d'autres termes, dans ce mode de réalisation avantageux, si la composition selon l'invention est sous forme liquide, ladite composition comprendra de 10⁴ à 10¹¹ colonies bactériennes de *Bacillus* par mL de composition, et cela pour chacune des souches lorsque la composition comprend au moins deux souches.

Dans l'invention, de 10⁴ à 10¹¹ colonies bactériennes signifie : environ 10⁴, environ 5. 10⁴, environ 10⁵, environ 5.10⁵, environ 10⁶, environ 5.10⁶, environ 10⁷, environ 5.10⁷, environ 10⁸, environ 5.10⁸, environ 10⁹, environ 5.10⁹, environ 10¹⁰, environ 5.10¹⁰ ou environ 10¹¹ colonies bactériennes.

L'homme du métier sait aisément comment déterminer ce nombre de bactéries, notamment par comptage soit manuel (en utilisant une lame de Malassez) ou en utilisant un compteur de cellule automatique, ou par dilution puis ensemencement sur gélose et comptage des colonies.

Encore plus avantageusement, l'invention concerne l'utilisation telle que définie précédemment, où les souches de *Bacillus subtilis* sont sous forme sporulée et/ou sous forme végétative.

Cela signifie que si la composition comprend une seule souche, cette souche peut être sous forme sporulée ou sous forme végétative ou un mélange de forme sporulée et de forme végétative. Par contre si la composition comprend au moins deux souches, ces souches peuvent être toutes les deux sous forme sporulées, ou sous forme végétative, ou l'un d'entre elle est sous forme végétative et l'autre est sous forme sporulée, ou les deux sont présentes sous les deux formes végétative et sporulée. Il en va de même lorsque la composition comprend 3 ou 4 souches susmentionnées.

Ladite mycotoxine est produite par un champignon appartenant au groupe constitué des champignons des genres *Alternaria, Aspergillus, Byssochkamys, Claviceps, Fusarium, Gibberella, Halosarpheia, Penicillium, Phoma, Pyricularia,* et *Verticillium,* et est choisie parmi les aflatoxines B1, B2, G1 et G2, la déoxynivalénol (ou nivalénol), la zéaralénone, et la fumonisine B1.

Les champignons susmentionnés, sans être limitatifs, sont capables de produire des mycotoxines qui peuvent être dégradées par la composition selon l'invention, c'est-à-dire par les au moins quatre souches distinctes de *Bacillus subtilis.* L'un des avantages de la composition selon l'invention par rapport à l'art antérieur, est que la composition n'est pas limitée à une mycotoxine, mais est capable de dégrader quasiment toutes ou toutes les mycotoxines.

Les aflatoxines constituent un groupe de 18 composés structurellement proches (un assemblage d'une coumarine et de 3 furannes). Elles sont produites par *Aspergillus flavus, Aspergillus parasiticus* et *Aspergillus nomius.* Parmi les plus courantes, on trouve l'AFB1, l'AFB2, l'AFM1, l'AFG1 et l'AFG2.

Les DON (déoxinivalénol) sont produits par différents contaminants fongiques du genre *Fusarium* (*F. graminearum, F. culmorum, F. roseum,* ...) qui contaminent les grains de blé, d'orge, d'avoine ou de maïs et même du riz lors de la floraison.

Le zéaralénone est une mycotoxine produite par certaines espèces de champignons du sol, les *Fusarium,* qui peuvent coloniser certains végétaux (notamment les graminées) en y produisant une maladie, la fusariose.

La fumonisine est une famille de mycotoxines émises par certaines espèces de champignons du sol, les *Fusarium,* (principalement *F*. *verticillioides* et *F. proliferatum*) qui peuvent coloniser certains végétaux (graminées notamment) en y produisant une maladie dite fusariose. On en connaît plusieurs variantes, dont la fumonisine B, très toxique, plutôt trouvée dans le maïs. Les fumonisines de type B, peuvent induire un oedème pulmonaire chez le porc, la leucoencéphalomalacie mortelle chez le cheval et une hypothèse est qu'elle pourrait aussi être une des causes de cancer de l'oesophage chez l'homme.

L'invention concerne avantageusement une méthode de dégradation des mycotoxines choisies parmi les aflatoxines B1, B2, G1 et G2, la déoxynivalénol (ou nivalénol), la zéaralénone, et la fumonisine B1, contenues dans un aliment destiné à la consommation animale comprenant une étape de mise en contact avec une composition comprenant ou consistant essentiellement en les souches NOL01, NOL02 et NOL03 lesdites souches étant déposées à la CNCM sous les numéros respectifs CNCM I - 4606, CNCM I-5043 et CNCM I - 4607 et éventuellement une étape d'inactivation après incubation, desdites souches de *Bacilllus subtilis,* notamment par la chaleur.

L'invention concerne en outre une méthode de décontamination d'aliment, de matière première ou de fourrage contaminé par au moins une mycotoxine, choisies parmi les aflatoxines B1, B2, G1 et G2, la déoxynivalénol (ou nivalénol), la zéaralénone, et la fumonisine B1, ladite méthode comprenant une étape de mise en contact de ladite denrée alimentaire ou dudit fourrage avec une composition comprenant ou consistant essentiellement en les souches NOL01, NOL02 et NOL03 lesdites souches étant déposées à la CNCM sous les numéros respectifs CNCM I - 4606, CNCM I-5043 et CNCM I - 4607

Cette méthode permet de décontaminer des produits alimentaires notamment en exposant, dans des conditions appropriées, des aliments, des matières premières ou des fourrages contaminés par une ou plusieurs mycotoxines avec la composition susmentionnée.

Les toxines seront alors, à l'issue de cette décontamination, dégradées, rendant aptes à la consommation sans risques lesdits aliments, matières premières et fourrages.

Avantageusement, l'invention concerne la méthode susmentionnée où ledit aliment, ladite matière première ou ledit fourrage est contaminé par au moins deux mycotoxines de structures différentes.

Dans un autre aspect, l'invention concerne une composition comprenant ou consistant essentiellement en les souches NOL01, NOL02 et NOL03, lesdites souches étant déposées à la CNCM sous les numéros respectifs CNCM I - 4606, CNCM I-5043 et CNCM I - 4607 pour son utilisation dans le cadre du traitement des animaux atteints d'une pathologie liée à l'ingestion d'au moins une mycotoxine, ou la prévention desdites pathologies, ladite mycotoxine étant choisie parmi les aflatoxines B1, B2, G1 et G2, la déoxynivalénol (ou nivalénol), la zéaralénone et la fumonisine B1.

Du fait de la capacité de dégradation des mycotoxines, la composition susmentionnée peut être utilisée à titre préventif contre les maladies ou symptômes développés suite à l'ingestion de mycotoxines. De la même manière, la composition selon l'invention peut être administrée à titre curatif chez des animaux intoxiqués par ladite mycotoxine.

Les effets qui sont susceptibles d'être développées suite à l'ingestion de mycotoxines sont multiples et sont par exemple des effets hépatotoxiques, neurotoxiques, mutagènes, tératogènes, cancérigènes, immunodépresseurs et/ou des perturbations endocriniennes.

La ou les bactéries contenues dans la composition selon l'invention peuvent être sous forme sporulée et/ou végétative. Sous cette forme, elles peuvent être administrées par voie orale (*per os*) directement via l'aliment, l'eau de boisson ou encore drogage (incorporation directement dans la bouche des animaux via une seringue)

Plus avantageusement, l'invention concerne la composition pour son utilisation susmentionnée, où ladite composition comprend de 10⁴ à 10¹¹ colonies bactériennes par mL ou par gramme de composition.

Avantageusement, l'invention concerne la composition susmentionnée pour son utilisation susmentionnée où lesdites pathologies sont liées à l'ingestion d'au moins deux mycotoxines de structures différentes.

L'invention sera mieux comprise à la lecture de l'exemple et des figures qui suivent.

### Brève description des dessins

**Fig. 1**

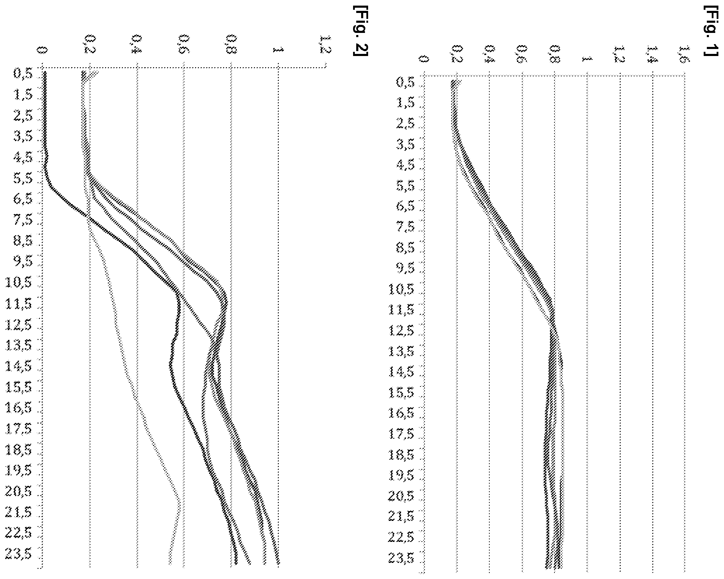

   **[****Fig. 1****]** représente un graphique de la croissance de NOL01 en fonction de différentes concentrations de ZEA dans le milieu (0, 0,1, 0,5 ,1 et 10 ppm). L'abscisse représentant le temps en heure et l'ordonnée l'absorbance de la solution corrélée à la croissance des bactéries.
**Fig. 2**
   **[****Fig. 2****]** représente un graphique de la croissance de NOL02 en fonction de différentes concentrations de ZEA dans le milieu (0, 0,1, 0,5, 1, 5 et 10 ppm). L'abscisse représentant le temps en heure et l'ordonnée l'absorbance de la solution corrélée à la croissance des bactéries.
**Fig. 3**

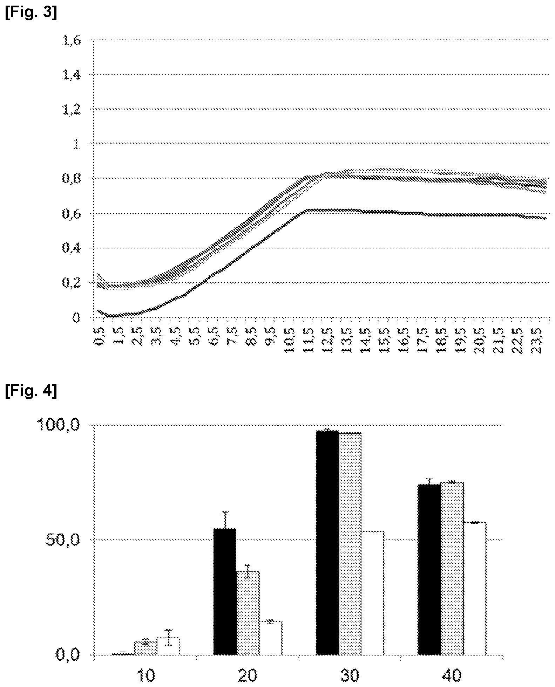

   **[****Fig. 3****]** représente un graphique de la croissance de NOL03 en fonction de différentes concentrations de ZEA dans le milieu (0, 0,1, 0,5, 1, 5 et 10 ppm). L'abscisse représentant le temps en heure et l'ordonnée l'absorbance de la solution corrélée à la croissance des bactéries.
**Fig. 4**
   **[****Fig. 4****]** représente le pourcentage de dégradation de la ZEA par les souches NOL01 (noir), NOL02 (blanc) et NOL03 (gris) en fonction de la température en degrés Celsius.
**Fig. 5**

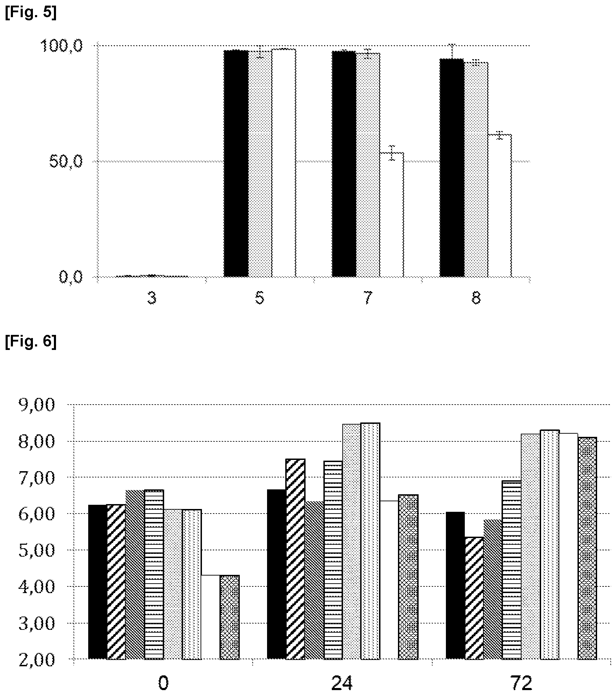

   **[****Fig. 5****]** représente le pourcentage de dégradation de la ZEA par les souches NOL01 (noir), NOL02 (blanc) et NOL03 (gris) en fonction du pH du milieu.
**Fig. 6**
   **[****Fig. 6****]** représente un histogramme montrant le nombre de bactéries (log CFU/mL) en fonction du temps (0, 16 et 72h) de bactéries NOL01 (barre noire), NOL02 (barre gris foncé), NOL03 (barre gris clair) et NOL04 (barre blanche) cultivées sans toxine ZEA, et de bactéries NOL01 (barre avec hachures diagonales), NOL02 (barre avec hachures horizontales), NOL03 (barre avec hachures verticales) et NOL04 (barre avec points) cultivées avec 1µg/ML de toxine ZEA.
**Fig. 7**

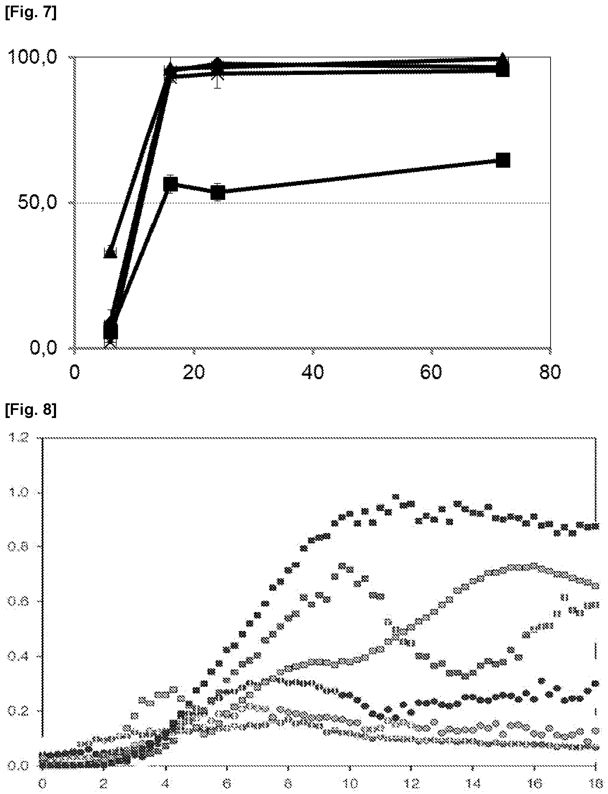

   **[****Fig. 7****]** est un graphique montrant le pourcentage de réduction de toxine ZEA au cours du temps (en heures) pour les souches NOL01 (losanges), NOL02 (carrés), NOL03 (triangles) et NOL04 (croix).
**Fig. 8**
   **[****Fig. 8****]** représente un graphique de la croissance des bactéries selon l'invention NOL01 (gris foncé), NOL02 (gris clair) et NOL03 (noir) en présence de ZEA et en fonction du milieu : milieu riche TSB (point carré), milieu minimal MM (point rond). L'abscisse représentant le temps en heure et l'ordonnée, l'absorbance de la solution corrélée à la croissance des bactéries (OD₆₀₀ₙₘ).

### EXEMPLES

### Exemple 1 - Tests in vitro

Les inventeurs ont testé les propriétés de réduction de la concentration en mycotoxine des souches bactériennes NOL01, NOL02 et NOL03 et NOL04 sur la ZEA (zéaralénone).

### 1- Effet de la présence de toxines sur la croissance des cellules

Les toxines ZEA utilisées pour cet essai sont fournies par Sigma-Aldrich (Milan, Italie) dont la pureté est supérieure à 99%. Les toxines sont dissoutes dans de l'acétonitrile à 1mg/mL puis filtrées sur un tube de filtration à membrane de cellulose (RC/G) de 0,2µm (LABOCHEM Science S.r.l., ITALY). Un volume approprié de solution standard de ZEA est ajouté à du milieu TBS pour obtenir la concentration finale souhaitée (1 µg/mL). Les différentes souches bactériennes ont été cultivées dans un milieu de culture TSB en présence ou non de 1µg/mL de ZEA à 30°C, avec une agitation à 150 rpm, en conditions aérobies.

Les données de culture sont représentées sur la figure 6. Ces résultats montrent que les souches NOL01, NOL02, NOL03 et NOL04 se développent aussi bien voire plus rapidement en présence de ZEA à une concentration de 1µg/mL de milieu.

Ensuite, différentes concentrations de ZEA dans le milieu ont été testées et les résultats ont montré que les souches NOL01, NOL02 et NOL03 se sont développées de manière équivalente dans toutes les concentrations de ZEA testées comme le montrent les figures 1 à 3.

Enfin, différentes conditions de pH et de température ont également été testées. Les résultats obtenus montrent que NOL01, NOL02 et NOL03 ont une activité de réduction de la ZEA à 10, 20 et 30°C (Figure 4) ainsi qu'aux pH 5, 7 et 8 (Figure 5).

Ces résultats montrent que les souches de Bacillus subtilis de l'invention ont la capacité de se développer en présence de fortes concentrations de mycotoxines, et que leur propriété de réduction de la concentration en mycotoxine est conservée dans de nombreuses conditions notamment de pH et température.

### 2- Mesure de la quantité de toxines après culture

Suite aux différentes cultures susmentionnées, les inventeurs ont testé la présence de toxines.

Afin de mesurer la quantité de toxine, les inventeurs ont utilisé les kits d'absorption DonTest^{™} et ZearalaTest^{™} (VICAM/Waters Corporation, Milford, MA, USA).

0,5 mL de surnageant de chaque culture est mélangé avec 1,5 mL de tampon phosphate (PBS) et appliqué sur les colonnes correspondantes pour adsorption de la toxine, à la vitesse d'une goutte par seconde. Les colonnes sont ensuite lavées avec 5mL d'eau distillée à la même vitesse.

Les toxines sont ensuite éluées avec 2mL de méthanol, et les éluats sont séchés à 50°C. Les produits secs sont ensuite repris dans un mélange de 250 µL H₂O:MeOH 85:15, avant analyse par spectrométrie de masse LC.

Pour la détection de la toxine ZEA, les inventeurs ont utilisé le dispositif Agilent 1100 HPLC system. Les conditions sont les suivantes :
- colonne analytique : Phenomenex ^{®} PFP, (100 mm × 4,6 mm, 2,6 µm), 40°C ;
- phase mobile : Eau+Méthanol+Acétonitrile 50+25+25 (v/v/v) ISOCRATIC ;
- débit : 0,800 mL/min ;
- temps de rétention : 6,7 min ;
- Fluorescence 274 nm λ(ex) et 440 nm λ(em).

Les résultats obtenus pour la toxine ZEA sont représentés dans le tableau suivant, et à la figure 3 :

**[Table 3]**

| ***temps (h)*** | | **Pourcentage de réduction de ZEA*** | | | |
|---|---|---|---|---|---|
| | | **NOL01** | **NOL02** | **NOL03** | **NOL04** |
| | 6 | 8,7±4,6 | 6,0±0,0 | 33,5±1,9 | 2,2±0,1 |
| | 16 | 95,5±1,5 | 57,0±3,1 | 96,1±4,2 | 93,2±1,7 |
| | 24 | 97,7±0,6 | 53,6±3,0 | 96,6±1,9 | 94,4±4,9 |
| | 72 | 96,9±0,6 | 64,8±2,1 | 99,4±1,1 | 95,5±1,0 |

| | | | | | |
|---|---|---|---|---|---|
| * moyenne des % obtenus sur 3 études indépendantes et écart type | | | | | |

Ces résultats montrent une réduction de la ZEA presque totale (plus de 94%) au bout de 24h avec NOL01, NOL03 et NOL04 et à plus de 50% pour NOL02.

Pour confirmer ces résultats, les inventeurs ont répété les expériences pour la toxine ZEA. Les résultats sont indiqués dans le tableau suivant :

**[Table 4]**

| **temps (h)** | **Pourcentage de réduction de ZEA*** | | | |
|---|---|---|---|---|
| | **NOL01** | **NOL02** | **NOL03** | **NOL04** |
| 24* | 97,7±0,6 | 53,6±3,0 | 96,6±1,9 | 94,4±4,9 |
| 24** | 95,7±2,5 | 56,5±2,5 | 91,3±1,4 | 91,8±0,8 |
| *Valeur P* | *0,238* | *0,264* | *0,415* | *0,090* |

| | | | | |
|---|---|---|---|---|
| * moyenne des % obtenus sur 3 études indépendantes et écart type | | | | |

Dans la mesure où la valeur P est supérieure à 0,05, les résultats sont considérés comme répétables. * : première série de tests, ** : seconde série de tests.

### 3- Test d'adsorption sur les bactéries

Les inventeurs ont ensuite testé si les toxines sont adsorbées sur les bactéries.

Pour ce faire, les cultures de bactéries après 24h en présence de ZEA ont été traitées de la manière suivante :
Les culots bactériens sont repris dans 1mL d'un mélange acétonitrile:eau (90 :10 ; v/v), et les mélanges sont agités vigoureusement à l'aide d'un vortex, et enfin centrifugés pendant 20 min à 14000g. Les surnageants obtenus sont ensuite séchés à 50°C et les résidus secs sont repris dans 1mL d'un mélange eau :méthanol 85 :15 v/v pour une analyse par spectrométrie comme détaillé ci-dessus.

Les résultats de désorption de ZEA sont indiqués dans le tableau suivant :

**[Table 5]**

| **Souche** | **Pourcentage de ZEA désorbée*** |
|---|---|
| (NOL01) | 3.2 |
| (NOL02) | 11.9 |
| (NOL03) | 4.0 |
| (NOL04) | 4.4 |

| | |
|---|---|
| * moyenne des % obtenus sur 3 études indépendantes et écart type | |

Ces résultats suggèrent que, au vu de la faible quantité de ZEA désorbée, que la toxine est soit internalisée dans les bactéries, soit dégradée par lesdites bactéries.

### 4- Test de dégradation par les bactéries

Afin de tester l'hypothèse de la dégradation des bactéries, les inventeurs ont testé la présence de toxine directement dans les bactéries.

Pour cela, à partir de bactéries cultivées pendant 24h avec 1µg/mL de ZEA, les culots bactériens ont été repris dans 1mL d'un mélange acétonitrile:eau (90 :10 ; v/v) et lysées par sonication. Les conditions de sonication sont les suivantes : diamètre de la sonde : 3,175 mm, fréquence : 20 kHz, amplitude: 50%, sonication : 15 sec puis arrêt 15 sec (6 fois), durée de sonication 90 sec au total (sans les temps de pause), température 4°C.

Après sonication, les échantillons sont centrifugés 20min à 14000g. Les surnageants obtenus sont ensuite séchés à 50°C et les résidus secs sont repris dans 1mL d'un mélange eau :méthanol 85 :15 v/v pour une analyse par spectrométrie comme détaillé ci-après. Dispositif : Aquity UPLC^{®} BEH C18 (100 mm × 2,1 mm, 1,7 µm) - 50°C ; Absorbance UV à 220 nm ; Fluorescence : 274 nm λ(ex) et 440 nm λ(em). Le temps de rétention de la ZEA était de 11,5 min.

Les résultats de ZEA extraite des différents culots sont les suivants :

**[Table 6]**

| **Souche** | **Pourcentage d'extraction de ZEA*** |
|---|---|
| (NOL01) | 2,7 |
| (NOL02) | 0,1 |
| (NOL03) | 0,0 |
| (NOL04) | 1,4 |

| | |
|---|---|
| * moyenne des % obtenus sur 3 études indépendantes et écart type | |

Ces résultats montrent que la ZEA n'est pas détectable, même lorsque les bactéries sont lysées, signifiant que la mycotoxine est dégradée par les bactéries.

### 5- Culture en conditions anaérobies

Les Inventeurs ont également testé les conditions de croissance bactériennes selon le 1- mais en absence d'oxygène.

Ils ont ensuite mesuré la dégradation de la mycotoxine ZEA dans ces conditions, selon les protocoles décrits ci-dessus.

Les résultats sont regroupés dans le tableau suivant :

**[Table 7]**

| **Type d' incubation** | | **Pourcentage de réduction de ZEA**** | | | |
|---|---|---|---|---|---|
| | | **NOL01** | **NOL02** | **NOL03** | **NOL04** |
| | *aerobie** | 97,7±0,6 | 53,6±3,0 | 96,6±1,9 | 94,4±4,9 |
| | *anaerobie** | 92,9±1,4 | 44,5±5,0 | 92,6±2,8 | 90,3±0,5 |
| | *Valeur P* | *0,259* | *0,080* | *0,530* | *0,121* |

| | | | | | |
|---|---|---|---|---|---|
| * les tests ont été réalisés après 24h de culture en présence de 1µg/ml de mycotoxine. ** moyenne des % obtenus sur 3 échantillons et écart type | | | | | |

Ces résultats montrent que, quelle que soit la condition de culture (présence ou absence d'oxygène), les bactéries sont capables de dégrader les mycotoxines.

### 6- Culture en milieux minimaux

Les inventeurs ont également testé les conditions de croissance bactériennes selon le 1- mais dans un milieu minimal c'est-à-dire un milieu appauvri, non enrichi (MM), sans apport de carbone autre que celui apporté par les mycotoxines, dont la composition est la suivante pour 1L de milieu de culture :

**[Table 8**

| **Composé** | **Quantité** |
|---|---|
| K₂HPO₄ | 2.72 g |
| KH₂PO₄ | 1g |
| Na₂SO₄ | 0.284g |
| NaNO₃ | 0.17g |
| KCl 0.15g | |
| CaCl₂ X 6H₂O | 22 mg |
| MnCl₂ X 4H₂O | 15 mg |
| FeCl₃ X 6H₂O | 2.16mg |
| MgCl₂ X 6H₂O | 25mg |
| NH₄Cl | 1g |
| Yeast extract | 0.05% |
| Eau | qsp |

Ces résultats montrent que les souches de *Bacillus subtilis* de l'invention ont la capacité de se développer dans un milieu minimal MM, même si le niveau de croissance est moindre que dans un milieu riche TSB.

Suite aux différentes cultures susmentionnées et à des cultures supplémentaires réalisées selon 1- mais en présence de 20 µg/mL de ZEA en milieu MM, en présence de 1 µg/mL de ZEA en condition anaérobie dans un milieu MM, dans un milieu minimal ré-enrichi en NOs (MMN) et dans un milieu minimal ré-enrichi en NOs et en glucose (MMNG), les inventeurs ont testé la présence de toxines selon 2- à t=72h.

Les résultats obtenus pour la toxine ZEA sont représentés dans le tableau suivant :

**[Table 9]**

| **Condition** | | **Milieu** | **ZEA** | **Pourcentage de réduction de ZEA*** | | |
|---|---|---|---|---|---|---|
| | | | | **NOL01** | **NOL02** | **NOL03** |
| | Aérobie | TSB | 1 µg/mL | 96,9 | 64,8 | 99,4 |
| | | MM | 1 µg/mL | 100 | 100 | 100 |
| | | | 20 µg/mL | 99,7 | 48,9 | 99 |
| | Anaérobie | TSB | 1 µg/mL | 92,9 | 44,5 | 92,3 |
| | | MM | 1 µg/mL | 32,5 | 24,1 | 32,5 |
| | | MMN | 1 µg/mL | 23 | 20,4 | 80,1 |
| | | MMNG | 1 µg/mL | 98,4 | 80,3 | 96,4 |

| | | | | | | |
|---|---|---|---|---|---|---|
| * moyenne des % obtenus sur 3 échantillons | | | | | | |

Ces résultats montrent, en condition aérobie, une réduction de la ZEA en milieu minimal MM comparable à celle observée en milieu riche TSB, quelle que soit la concentration de ZEA de départ.

En condition anaérobie, un même niveau de dégradation des mycotoxines qu'en milieu riche TSB est atteint par les trois souches selon l'invention, testées en milieu minimal ré-enrichi en NOs et en glucose (MMNG). Même conclusion pour NOL03 en milieu minimal ré-enrichi en NO₃ (MMN).

Ces résultats montrent que, quel que soit le milieu de culture (riche, minimal ou minimal ré-enrichi), les bactéries selon l'invention sont capables de dégrader les mycotoxines, avec plus d'efficacité dans les milieux riches et minimaux ré-enrichis.

### 7- Réduction de mycotoxines autres que la ZEA

Les inventeurs ont également réalisé des cultures de bactéries selon l'invention selon le 1- mais en présence de mycotoxines autres que la ZEA, puis mesuré la quantité de toxine après culture selon le 2- avec des kits d'absorption appropriés pour chacune des mycotoxines testées, à t=72h.

**[Table 10]**

| | **Pourcentage de réduction*** | | |
|---|---|---|---|
| | **NOL01** | **NOL02** | **NOL03** |
| ZEA | 96,9 | 64,8 | 99,4 |
| FB₁ | 4,7 | 18,7 | 8,8 |
| OTA | 7,3 | 10,0 | 15,9 |
| T-2 | 7,2 | 1,0 | 4,2 |

| | | | |
|---|---|---|---|
| * moyenne des % obtenus sur 3 échantillons | | | |

Ces résultats montrent, en condition aérobie, une capacité de réduction de NOL01 sur ZEA, OTA, et T-2, l'effet observé sur ces deux dernières mycotoxines étant cependant plus faible. De même, ils montrent une capacité de réduction de NOL02 et de NOL03 sur ZEA, FB₁ et OTA, l'effet observé sur ces deux dernières mycotoxines étant cependant plus faible.

### 8- Test d'efficacité des bactéries NOL01, NOL02 et NOL03 mises en association

Les inventeurs ont également réalisé des cultures de bactéries selon l'invention selon le 1- en présence de ZEA, AFB₁ et FB₁, en milieu riche (TSB) ou minimal ré-enrichi en NOs et en glucose (MMNG), mais avec un inoculum contenant 1µg/mL d'une association des trois bactéries selon l'invention NOL01, NOL02, NOL03 mises en oeuvre en proportion égale.

**[Table 11]**

| | | | **Pourcentage de réduction* à t=72h** | | | |
|---|---|---|---|---|---|---|
| | | | **NOL01** | **NOL02** | **NOL03** | **POOL** |
| Aérobie | TSB | ZEA | 96,9 | 64,8 | 99,4 | 100 |
| Anaérobie | MMNG | ZEA | 98,4 | 80,3 | 96,4 | Non testé |
| | | AFB₁ | 1,6 | 0,5 | 0,7 | 11,7 |
| | | FB₁ | 3,0 | 4,4 | 2,0 | 18,1 |

| | | | | | | |
|---|---|---|---|---|---|---|
| * moyenne des % obtenus sur 3 échantillons | | | | | | |

Ces résultats mettent en évidence une capacité de réduction de la présence de mycotoxines ZEA par les bactéries NOL01, NOL02 et NOL03 mises en association que ce soit en condition aérobie en milieu riche (TSB), ou en condition anaérobie en milieu minimal ré-enrichi en NO₃ et en glucose (MMNG). On constate également une capacité de réduction de la présence des AFB₁ et FB₁ de ce pool en condition anaérobie et milieu MMNG, bien que plus réduite.

### 9- Mesure d'efficacité contre les métabolites de la ZEA

Les inventeurs ont vérifié la capacité de réduction des mycotoxines des souches NOL01, NOL02 et NOL03 selon l'invention, selon 1- mais sur les métabolites de la ZEA, en condition aérobie en milieu minimal (MM) et en condition anaérobie en milieu minimal ré-enrichi en NOs et en glucose (MMNG).

**[Table 12]**

| | **Pourcentage de réduction* à t=72h** | | | | | |
|---|---|---|---|---|---|---|
| | **Aérobie** | | | **Anaérobie** | | |
| | **MM** | | | **MMNG** | | |
| | NOL01 | NOL02 | NOL03 | NOL01 | NOL02 | NOL03 |
| ZEA | 100 | 98,9 | 99,0 | 100 | 96,3 | 96,3 |
| α-ZOL | 100 | 98,4 | 99,0 | 100 | 56,6 | 100 |
| β-ZOL | 100 | 96,6 | 99,0 | 100 | 47,0 | 100 |
| α-ZAL | 99,0 | 85,3 | 96,0 | 100 | 32,7 | 79,4 |
| β -ZAL | 98,9 | 80,0 | 93,0 | 100 | 44,5 | 87 |
| ZAL | 99,0 | 90,8 | 85,7 | 100 | 20,2 | 68,6 |

| | | | | | | |
|---|---|---|---|---|---|---|
| * moyenne des % obtenus sur 3 études indépendantes et écart type | | | | | | |

Ces résultats confirment que, quel que soit les conditions de culture (présence ou absence d'oxygène, milieu riche, minimal ou minima ré-enrichi), les bactéries selon l'invention sont capables de dégrader les ZEA ainsi que ses métabolites.

### Exemple 2 - Test in vivo chez le porc

Les inventeurs ont également testé les bactéries selon l'invention en conditions *in vivo,* chez le porc, afin de démontrer leur efficacité chez l'animal.

### 1- Protocole

Les inventeurs ont réalisé un essai d'alimentation de 21 jours en station expérimentale sur 64 porcelets sevrés répartis en 2 tests, l'un sur ZEA, l'autre sur DON (soit 32 porcelets par test).

Les régimes suivants ont été appliqués pour chacun de ces tests (soit 8 porcelets par régime) :
(1) aliment non contaminé sans additif
(2) aliment non contaminé + additif
(3) aliment contaminé sans additif
(4) aliment contaminé + additif

Pour les régimes (3) et (4), l'aliment distribué à l'animal était contaminé artificiellement à hauteur de 0.1 mg ZEA/kg d'aliment complet à 12% d'humidité, soit la teneur maximale recommandée par la réglementation européenne selon la Recommandation de la Commission européenne n°2006/576/CE.

Pour le test sur DON, les 4 mêmes régimes ont été appliqués, avec une contamination artificielle de l'aliment distribué à hauteur de 0.9 mg DON/kg d'aliment complet à 12% d'humidité pour les régimes (3) et (4).

La quantité de mycotoxines a été analysée en début d'essai et une bonne homogénéité de la contamination de l'aliment distribué a été confirmée.

L'additif tel qu'ajouté correspondait à l'association des 3 souches selon l'invention NOL01, NOL2 et NOL3 mises en oeuvre en proportion égale.. Cet additif, sous forme de poudre, a été ajouté à l'aliment distribué après contamination artificielle en mycotoxines et avant distribution aux animaux et en quantité suffisante pour atteindre 10⁹ colonies bactériennes par kg d'aliment.

Environ 1,5 kg d'aliment sont distribués par jour par porcelet.

Les animaux étaient répartis en 1 animal par cage métabolique pour le test sur ZEA, et 1 animal par enclos pour le test sur DON.

Une période d'adaptation de 3 semaines avant le lancement des régimes tests a été respectée afin d'éviter un stress des animaux lors du passage en post-sevrage et d'acclimater ceux qui étaient en cages métaboliques pour le test sur ZEA.

A l'issue de cette période d'adaptation de 3 semaines, les tests ont débuté et ont duré 3 semaines (21 jours).

### 2- Analyses et paramètres zootechniques

Les échantillons suivants ont été récoltés tout au long de l'essai :
- Prise de sang à 0, 7, 14 et 21 jours sur tous les animaux,
- Récolte de faeces et d'urine entre le 17^{ème} et le 21^{ème} jours pour le test sur ZEA,
- Récolte de faeces à 21 jours, à l'abattage pour le test sur DON,
- Prise d'échantillons de tissus à 21j : foies, reins, muscles, organes reproducteurs, tractus intestinaux.

Sur les échantillons récoltés, en priorité les échantillons récoltés en fin d'essai, les analyses suivantes ont été réalisées :
- Détection de ZEA et ses métabolites α-zearalenol et β-zearalenol dans le plasma, l'urine et les faeces récoltés sur les animaux du test sur ZEA,
- Détection de DON et ses métabolites dans le sérum récolté sur les animaux du test sur DON.

Pour les 2 tests, les paramètres zootechniques suivants ont été relevés : poids corporel, consommation alimentaire journalière moyenne, mortalité, morbidité. Pour le test sur ZEA, ont également été relevés les paramètres suivants : taille de la vulve et/ou degré d'inflammation de la vulve, poids des organes reproducteurs.

## Revendications

1. Utilisation *in vitro* ou *ex vivo* d'une composition comprenant ou consistant essentiellement en au moins la souche de *Bacillus subtilis* NOL03, la souche de *Bacillus subtilis* NOL01 et la souche de *Bacillus subtilis* NOL02,
- ladite souche de *Bacillus subtilis* NOL03 étant déposée à la CNCM sous le numéro CNCM I - 4607,
- ladite souche de *Bacillus subtilis* NOL01 étant déposée à la CNCM sous le numéro CNCM I - 4606, et
- ladite souche de *Bacillus subtilis* NOL02 étant déposée à la CNCM sous le numéro CNCM I - 5043, pour la dégradation d'au moins une mycotoxine,
ladite au moins une mycotoxine est choisie parmi la zéaralénone, les aflatoxines B1, B2, G1 et G2, la déoxynivalénol ou nivalénol, et la fumonisine B1.

2. Utilisation selon la revendication 1, pour la dégradation d'au moins deux mycotoxines de structures différentes.

3. Utilisation selon des revendications 1 ou 2, où ladite composition comprend de 10⁴ à 10¹¹ colonies bactériennes, les colonies bactériennes étant par mL ou g de composition.

4. Méthode de décontamination d'aliment, de matière première ou de fourrage contaminé par au moins une mycotoxine, ladite méthode comprenant une étape de mise en contact de ladite denrée alimentaire ou dudit fourrage avec une composition comprenant ou consistant essentiellement en la souche de *Bacillus subtilis* NOL03, la souche de *Bacillus subtilis* NOL01 et la souche de *Bacillus subtilis* NOL02,
- ladite souche de *Bacillus subtilis* NOL03 étant déposée à la CNCM sous le numéro CNCM I - 4607,
- ladite souche de *Bacillus subtilis* NOL01 étant déposée à la CNCM sous le numéro CNCM I - 4606, et
- ladite souche de *Bacillus subtilis* NOL02 étant déposée à la CNCM sous le numéro CNCM I - 5043,
ladite au moins une mycotoxine est choisie parmi les aflatoxines B1, B2, G1 et G2, la déoxynivalénol ou nivalénol, la zéaralénone et la fumonisine B1.

5. Méthode selon la revendication 4, où ledit aliment, ladite matière première ou ledit fourrage est contaminé par au moins deux mycotoxines de structures différentes.

6. Composition comprenant ou consistant essentiellement en la souche de *Bacillus subtilis* NOL03, la souche de *Bacillus subtilis* NOL01 et la souche de *Bacillus subtilis* NOL02,
- ladite souche de *Bacillus subtilis* NOL03 étant déposée à la CNCM sous le numéro CNCM I - 4607,
- ladite souche de *Bacillus subtilis* NOL01 étant déposée à la CNCM sous le numéro CNCM I - 4606, et
- ladite souche de *Bacillus subtilis* NOL02 étant déposée à la CNCM sous le numéro CNCM I - 5043,
pour son utilisation dans le cadre du traitement des animaux atteints d'une pathologie liée à l'ingestion d'au moins une mycotoxine, ou la prévention desdites pathologies,
ladite au moins une mycotoxine est choisie parmi les aflatoxines B1, B2, G1 et G2, la déoxynivalénol ou nivalénol, la zéaralénone et la fumonisine B1.

7. Composition pour son utilisation selon la revendication 6, où lesdites pathologies sont liées à l'ingestion d'au moins deux mycotoxines de structures différentes.

## Patentansprüche

1. *In-vitro-* oder Ex-vivo-Verwendung einer Zusammensetzung, umfassend oder im Wesentlichen bestehend aus mindestens dem Stamm NOL03 von *Bacillus subtilis,* dem Stamm NOL01 von *Bacillus subtilis* und dem Stamm NOL02 von *Bacillus subtilis,*
- wobei der Stamm NOL03 von *Bacillus subtilis* bei der CNCM unter der Nummer CNCM I - 4607 hinterlegt ist,
- wobei der Stamm NOL01 von *Bacillus subtilis* bei der CNCM unter der Nummer CNCM I - 4606 hinterlegt ist, und
- wobei der Stamm NOL02 von *Bacillus subtilis* bei der CNCM unter der Nummer CNCM I - 5043 hinterlegt ist,
für den Abbau von mindestens einem Mykotoxin,
wobei das mindestens eine Mykotoxin aus Zearalenon, Aflatoxinen B1, B2, G1 und G2, Deoxynivalenol oder Nivalenol und Fumonisin B1 ausgewählt ist.

2. Verwendung nach Anspruch 1 für den Abbau von mindestens zwei Mykotoxinen mit unterschiedlichen Strukturen.

3. Verwendung nach Anspruch 1 oder 2, wobei die Zusammensetzung 10⁴ bis 10¹¹ Bakterienkolonien umfasst, wobei die Bakterienkolonien pro ml oder g der Zusammensetzung sind.

4. Verfahren zum Dekontaminieren eines Nährmittels, Rohmaterials oder Futters, das mit mindestens einem Mykotoxin kontaminiert ist, das Verfahren umfassend einen Schritt eines Inberührungbringens des Nahrungsmittels oder des Futters mit einer Zusammensetzung, umfassend oder im Wesentlichen bestehend aus dem Stamm NOL03 von *Bacillus subtilis,* dem Stamm NOL01 von *Bacillus subtilis* und dem Stamm NOL02 von *Bacillus subtilis,*
- wobei der Stamm NOL03 von *Bacillus subtilis* bei der CNCM unter der Nummer CNCM I - 4607 hinterlegt ist,
- wobei der Stamm NOL01 von *Bacillus subtilis* bei der CNCM unter der Nummer CNCM I - 4606 hinterlegt ist, und
- wobei der Stamm NOL02 von *Bacillus subtilis* bei der CNCM unter der Nummer CNCM I - 5043 hinterlegt ist,
wobei das mindestens eine Mykotoxin aus Aflatoxinen B1, B2, G1 und G2, Deoxynivalenol oder Nivalenol, Zearalenon und Fumonisin B1 ausgewählt ist.

5. Verfahren nach Anspruch 4, wobei das Nährmittel, das Rohmaterial oder das Futter mit mindestens zwei Mykotoxinen mit unterschiedlichen Strukturen kontaminiert ist.

6. Zusammensetzung, umfassend oder im Wesentlichen bestehend aus dem Stamm NOL03 von *Bacillus subtilis,* dem Stamm NOL01 von *Bacillus subtilis* und dem Stamm NOL02 von *Bacillus subtilis,*
- wobei der Stamm NOL03 von *Bacillus subtilis* bei der CNCM unter der Nummer CNCM I - 4607 hinterlegt ist,
- wobei der Stamm NOL01 von *Bacillus subtilis* bei der CNCM unter der Nummer CNCM I - 4606 hinterlegt ist, und
- wobei der Stamm NOL02 von *Bacillus subtilis* bei der CNCM unter der Nummer CNCM I - 5043 hinterlegt ist,
zur Verwendung in dem Rahmen der Behandlung von Tieren, die an einer Erkrankung leiden, die mit der Einnahme von mindestens einem Mykotoxin verbunden ist, oder bei der Vorbeugung dieser Erkrankungen,
wobei das mindestens eine Mykotoxin aus Aflatoxinen B1, B2, G1 und G2, Deoxynivalenol oder Nivalenol, Zearalenon und Fumonisin B1 ausgewählt ist.

7. Zusammensetzung zur Verwendung nach Anspruch 6, wobei die Erkrankungen mit der Einnahme von mindestens zwei Mykotoxinen mit unterschiedlichen Strukturen verbunden sind.

## Claims

1. In vitro or ex vivo use of a composition comprising of consisting essentially of at least *Bacillus subtilis* NOL03, *Bacillus subtilis* NOL01 and *Bacillus subtilis* NOL02 strains,
- said *Bacillus subtilis* NOL03 being deposited at CNCM under number CNCM I - 4607;
- said *Bacillus subtilis* NOL01 being deposited at CNCM under number CNCM I - 4606; and
- said *Bacillus subtilis* NOL02 being deposited at CNCM under number CNCM I - 5043;
for degrading at least one mycotoxin, said at least one mycotoxin being chosen from zearalenone, aflatoxines B1, B2, G1 and G2, deoxynivalenol or nivalenol and fumonisin B1.

2. Use according to claim 1, for degrading at least two structurally different mycotoxin.

3. Us according to claim 1 or 2, wherein said composition comprises from 10⁴ to 10¹¹ bacterial colonies, bacterial colonies being by mL or g of the composition.

4. A method for decontamination of a food, raw material of fodder contaminated by at least a mycotoxin, said method comprising a step of contacting said food or fodder with a composition comprising or consisting essentially of at least *Bacillus subtilis* NOL03, *Bacillus subtilis* NOL01 and *Bacillus subtilis* NOL02 strains,
- said *Bacillus subtilis* NOL03 being deposited at CNCM under number CNCM I - 4607;
- said *Bacillus subtilis* NOL01 being deposited at CNCM under number CNCM I - 4606; and
- said *Bacillus subtilis* NOL02 being deposited at CNCM under number CNCM I - 5043;
said at least one mycotoxin being chosen from les aflatoxins B1, B2, G1 and G2, deoxynivalenol or nivalenol, zearalenone and fumonisin B1.

5. Method according to claim 4, wherein said food, raw material of fodder is contaminated by at least two structurally different mycotoxins.

6. Composition comprising of consisting essentially of NOL03 *Bacillus subtilis* strain, NOL01 *Bacillus subtilis* strain and NOL02 *Bacillus subtilis* strain ,
- said *Bacillus subtilis* NOL03 being deposited at CNCM under number CNCM I - 4607;
- said *Bacillus subtilis* NOL01 being deposited at CNCM under number CNCM I - 4606; and
- said *Bacillus subtilis* NOL02 being deposited at CNCM under number CNCM I - 5043;
for its use for treating animals afflicted by pathologies linked to ingestion of at least one mycotoxin, or the prevention of said pathologies,
said at least one mycotoxin being chosen from les aflatoxins B1, B2, G1 and G2, deoxynivalenol or nivalenol, zearalenone and fumonisin B1.

7. Composition for its use according to claim 6, wherein said pathologies are linked to ingestion of at least two structurally different mycotoxins.
